# EUROPEAN PATENT SPECIFICATION

(11) **EP 3 840 819 B1**
(45) Date of publication and mention of the grant of the patent: **19.11.2025**
(21) Application number: 19779583.4
(22) Date of filing: 19.08.2019
(51) Int. Cl.: A61M 35/00

(54) **APPLICATOR**
APPLIKATOR
APPLICATEUR

(30) Priority: 21.08.2018 US 201862720682 P; 11.07.2019 US 201962872838 P
(43) Date of publication of application: 30.06.2021
(73) Proprietor: Solventum Intellectual Properties Company, Maplewood, MN 55144 (US)
(72) Inventor: DOMBROWSKI, Alan R., Saint Paul, Minnesota 55133-3427 (US); ASMUS, Robert A., Saint Paul, Minnesota 55133-3427 (US)
(74) Representative: Mathys & Squire
(86) International application number: PCT/IB2019/056983
(87) International publication number: WO 2020/039330

(56) References cited:
- EP-A1- 0 040 959
- EP-B1- 0 040 959
- WO-A1-2015/042021
- US-A- 5 791 801
- US-A1- 2007 147 946
- US-A1- 2010 168 638

## Description

### TECHNICAL FIELD

The present disclosure relates to an applicator for applying a liquid to a surface. In particular the present disclosure relates to an applicator including a frangible ampoule.

### BACKGROUND

Barrier products are used to protect the skin of patients who suffer from a variety of conditions including, for example, urinary and fecal incontinence, skin occlusion, ileostomy and colostomy. The presence of high moisture and corrosive enzymes from intestinal fluids particularly can lead to devastating breakdown of the skin, which can lead to fungal infections and denuding and erosion of the skin. Some of the products which can be used to protect the skin from these challenges include cyanoacrylate monomer solutions, solutions that require special storage conditions due to their tendencies to degrade upon exposure to ambient conditions.

One method of maintaining the functionality of solutions including cyanoacrylate monomers is to seal such solutions in a container, such as a glass ampoule that can be crushed to release the solution shortly before use. Applicators for dispensing small aliquots of solution, *e.g.,* 0.5 mL to 2 mL, which are particularly desirable when the area to be covered is less than 30 square inches (200 cm²) typically employ glass ampoules placed into plastic tubes with foam attached to them and do not have levers for crushing the ampoule. To provide for a safe device, a suitably rigid and/or thick plastic wall material must be used to form the plastic tube, but since the tube is generally pinched between the fingers to activate, these devices may be difficult for users to activate, especially with a single hand.

### SUMMARY

In one aspect, provided is an applicator for applying a substance to a surface, the applicator comprising:
a hollow body, wherein the hollow body includes a distal portion, a proximal portion, an exterior surface, an interior surface, an activation region, and a filter;
a frangible ampoule, wherein the frangible ampoule contains the substance;
a cap, wherein the cap includes a proximal portion, a distal portion, an exterior surface, and an interior surface, and;
a spacer, wherein the spacer includes a distal end and a proximal end, wherein the frangible ampoule is located in the hollow body between the filter and the distal end of the spacer, wherein at least a portion of the frangible ampoule is located in the activation region, and wherein the spacer is located between the frangible ampoule and the cap.

Features and advantages of the present disclosure will be further understood upon consideration of the detailed description as well as the appended claims.

### BRIEF DESCRIPTION OF THE DRAWINGS

**FIG. 1** shows a perspective view of one embodiment of an applicator of the present disclosure.
**FIG. 2** shows an exploded view of the applicator of **FIG. 1****.**
**FIG. 3** shows a perspective view of the applicator body and head of the applicator of **FIG. 1****.**
**FIG. 4** shows a longitudinal cross-section of the applicator of **FIG. 1****.**
**FIG. 5** shows an axial view from the proximal end of the body of the applicator of **FIG. 1****.**
**FIG. 6** shows an axial cross-section of the applicator of **FIG.1** through the ampoule in the activation region.
**FIG. 6a** shows a longitudinal cross-section of another embodiment of the applicator of the present disclosure.
**FIG. 7** shows an axial view of the applicator head from the distal end of the applicator of **FIG. 1****.**
**FIG. 8** shows a perspective view of the cap and spacer according of the applicator of **FIG. 1****.**
**FIG. 8a** shows a perspective view of another embodiment of the cap and spacer according of the applicator of **FIG. 1**
**FIG. 9** shows a first cross-sectional view of the cap of **FIG. 8** where the channel is not visible.
**FIG. 10** shows a second cross-sectional view of the cap of **FIG. 8** where the channel is visible.
**FIG. 11** shows a perspective view of another embodiment of an applicator cap according to the present disclosure.
**FIG. 12** shows a cross-sectional view of another embodiment of an applicator cap and spacer according to the present disclosure.
**FIG. 13** shows an exploded view of an applicator including the cap and spacer of **FIG. 12****.**

Repeated use of reference characters in the specification and drawings is intended to represent the same or analogous features or elements of the disclosure. It should be understood that many other modifications and embodiments can be devised by those skilled in the art, which fall within the scope and spirit of the principles of the disclosure. The figures may not be drawn to scale.

### DETAILED DESCRIPTION

### Definitions:

The term "a", "an", and "the" are used interchangeably with "at least one" to mean one or more of the elements being described.

The term "and/or" means either or both. For example, "A and/or B" means only A, only B, or both A and B.

The terms "including," "comprising," and "having," and variations thereof, are meant to encompass the items listed thereafter, and equivalents thereof, as well as additional items.

The terms "polymer" and "polymeric material" refer to both materials prepared from one monomer such as a homopolymer or to materials prepared from two or more monomers such as a copolymer, terpolymer, or the like. Likewise, the term "polymerize" refers to the process of making a polymeric material that can be a homopolymer, copolymer, terpolymer, or the like. The terms "copolymer" and "copolymeric material" refer to a polymeric material prepared from at least two monomers.

The terms "longitudinal" and "axial" are used to refer to a direction or axis that is generally parallel to a central longitudinal axis of an applicator and generally parallel to the overall direction of substance flow.

The terms "lateral" and "transverse" are used to refer to a direction or axis that is perpendicular to the central longitudinal axis the longitudinal direction.

The terms "vertical" and "normal" are used to refer to a direction or axis that is normal to both the longitudinal and lateral directions (or axes).

The term "proximal" and "distal" are used to represent longitudinal or axial directions, relative to a user (such as, for example, a medical practitioner) using or holding the applicator. That is, the term "distal" is used to refer to the direction away from the medical practitioner (and toward a surface (such as, for example, a skin surface) to be treated, *i.e.,* to which the applicator will apply a substance); and the term "proximal" is used to refer to the direction toward the user (and away from the surface to be treated). For example, the distal end of an applicator is configured to be directed toward, or even pressed against, the surface to be treated, while the proximal end extends away from the surface and toward the user. Similarly, the distal end of any portion or component of an applicator is configured to be directed or oriented toward the surface to be treated and is oriented toward the distal end of the applicator or forms or defines the distal end of the applicator. In addition, the proximal end of any portion or component of an applicator is configured to be directed or oriented away from the surface to be treated and is oriented toward the proximal end of the applicator or forms or defines the proximal end of the applicator.

It is often desirable to apply substances to surfaces. In the medical field, single-use applicators may be used to apply medical products to the skin or other tissues. Medical preparations such as, for example, skin barrier products, antiseptics, adhesion enhancing products, adhesive tape trauma protectants, and pharmaceutical agents such as analgesics may be applied as, for example, liquid solutions containing one or more substances. These solutions may be applied with saturated sponges that are attached to a blade or held with forceps. These sponges are often saturated by soaking them in open pans of solution. Sometimes, sponges with attached handles are provided in a plastic or aluminum foil laminate pouch containing enough liquid to saturate the sponges. In some products the sponges are supplied dry in a sterile "kit" with the antiseptic solutions provided in relatively thin-walled polyethylene bottles. These bottles generally have wall thickness less than about 500 microns. While inexpensive, these techniques are messy and offer little control over inadvertent dripping of the solution into areas where it is undesired.

Alternatively, devices have been developed in an attempt to prevent solution dripping associated with these techniques, and to reduce the time required for application of the antiseptic solution. For example, liquid applicators that hold the liquid in a frangible ampoule and require additional elements to crush the ampoule and release the liquid have been developed. However, existing applicators are often complex to construct, may be difficult or cumbersome to use, and may deliver far more solution than is required for a given treatment, particularly when a relatively small area of tissue requires substance application.

Treatment of small areas of skin with a solution is needed in some situations, such as, for example, in the application of skin barrier products to persons with ostomies, including ileostomies and colostomies. Treatment of these smaller areas may present challenges not encountered when larger treatment areas are involved. For example, because the treatment area is small, an applicator containing a small volume of solution is preferred to minimize wasting what may be a costly solution. Moreover, for conditions such as ostomies, such products may be applied in a nonclinical setting by the person with the condition, creating a need for a simple and easy-to-use applicator that can reliably deliver less than 5 mL *(e.g.,* 0.2 - 2 mL) of a solution to a surface in need of treatment.

Applicators that can hold a small amount of substance (*e.g*., less than 5 mL), that allow for sterilization processes, *e.g*., ethylene oxide exposure, and that may be activated and ergonomically used with one hand are needed for certain applications. The present disclosure provides an applicator that, *inter alia,* includes a handle large enough to be ergonomic while still providing an ampoule suitable for containing a small amount of a substance, the applicator including a spacer that maintains the ampoule in an activation region of the applicator body so that the ampoule may be fractured while the applicator is held in a single hand.

In one aspect, the present disclosure provides an applicator for applying a substance to a surface. Generally, the applicator comprises an elongated hollow body comprising a distal portion, a proximal portion having an open end, an exterior surface, an interior surface, and a filter. The interior surface of the applicator defines an internal chamber suitable for receiving an ampoule. A first ampoule formed of a frangible material is located in the internal chamber proximal to the filter and contains a substance to be applied. A spacer having a distal end and a proximal end is provided, and a cap having a proximal portion, a distal portion, an exterior surface, and an interior surface is provided that attaches to the open end of the applicator and maintains the ampoule and spacer in their respective desired positions. The applicator includes a head and an elongated hollow body comprising a distal portion, a proximal portion having an open end, an exterior surface, an interior surface, and a filter. The interior surface of the applicator defines an internal chamber suitable for receiving an ampoule. A first ampoule formed of a frangible material may be located in the internal chamber proximal to the filter and contains a substance to be applied. A spacer having a distal end and a proximal end is provided, and a cap having a proximal portion, a distal portion, an exterior surface, and an interior surface is provided that attaches to the open end of the applicator and maintains the ampoule and spacer in their respective desired positions. The applicator further comprises an absorbent component that is capable of absorbing the contents of the frangible ampoule and facilitate applying the contents to the desired surface.

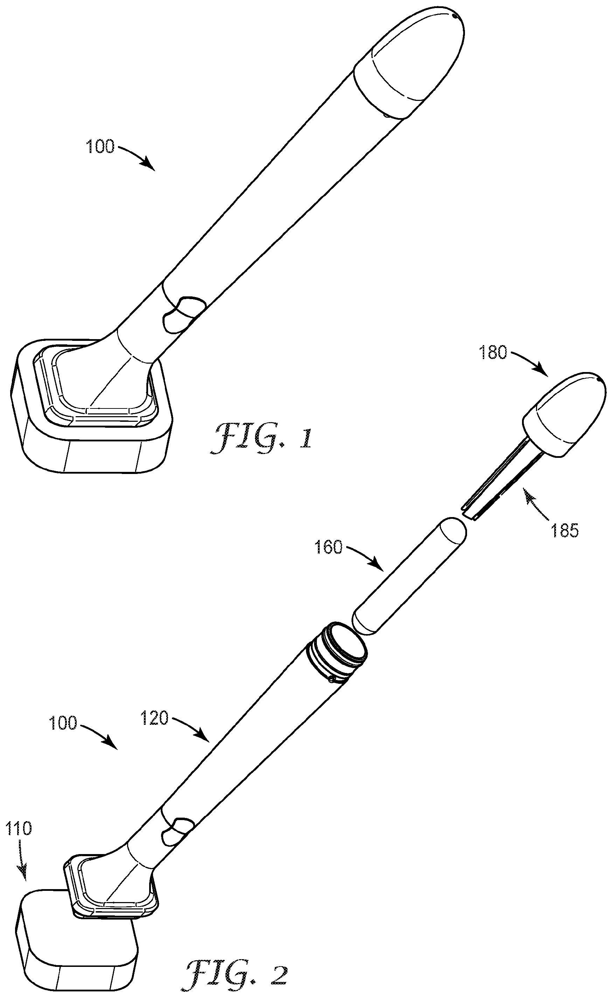

An exemplary applicator **100** according to one embodiment of the present disclosure is illustrated in **FIGS. 1-4****.** The applicator **100** may include an absorbent component **110,** a hollow body **120,** a frangible ampoule **160,** a cap **180,** and a spacer **185.**

The absorbent component **110** may be formed of any suitable porous substance such as, for example, a sponge, a woven material, a nonwoven material, a screen, a mesh, and combinations thereof. Materials suitable for use as the absorbent component **110** may include, for example, polyester polyurethane and polyester polyether open-cell foams. The absorbent component **110** may be attached to the head **140** at the distal end **123** of the hollow body **120** and may, among other functions, aid in control of the flow rate and distribution of the contents of the frangible ampoule **160** after the contents have been released from the frangible ampoule **160.**

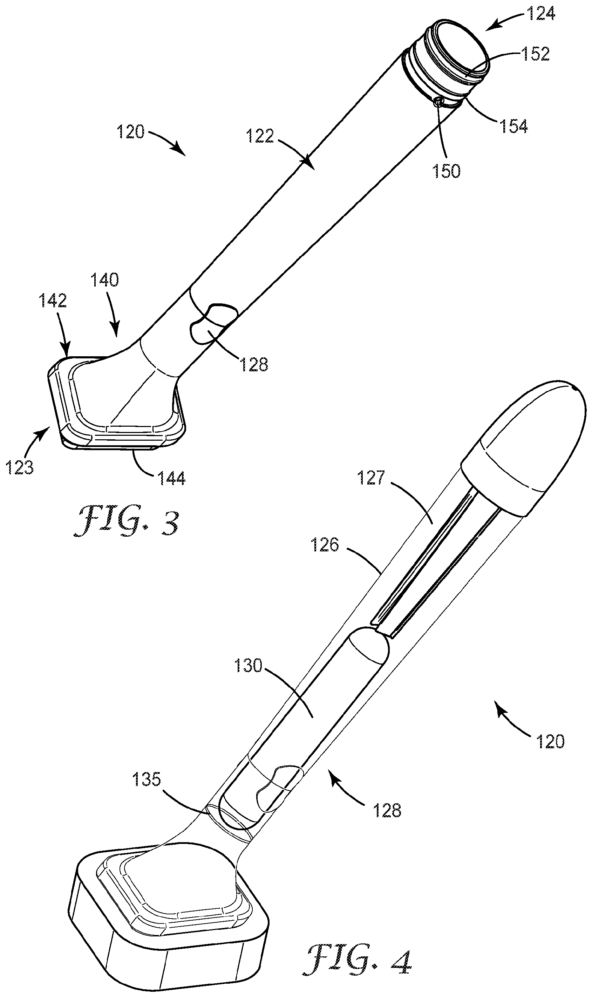

In some embodiments, the absorbent component **110** may be attached to the head **140** at the distal end **123** of the hollow body **120** by, for example, hot plate welding. Referring to **FIG. 3****,** absorbent component **110** may be attached to flange **142** by heating lip **144** to a suitable temperature and then bringing the heated lip **144** and the absorbent component **110** into contact under pressure. In other embodiments, the absorbent component **110** may be attached to hollow body **120** by other means, such as, for example, an adhesive, a spin weld, an ultrasonic weld, or combinations thereof.

The hollow body **120** includes a distal portion **123** and a proximal portion **124,** inner surface **126** and outer surface **127.** The hollow body **120** includes a handle section **122** that is generally elongated and tapered, e.g., the hollow body **120** may have a frustoconical geometry and may taper as shown along the longitudinal axis of the hollow body **120** from the proximal portion **124** to the distal portion **123** (*see* **FIGS. 1-4** and **13**) to facilitate gripping of the applicator **100** by the user and configured to retain the frangible ampoule **160.** The handle section **122** further includes activation region **128.** Application of a force to the activation region **128** desirably results in the fracture of the frangible ampoule **160** that is contained in the hollow body **120.** The force may be applied by, for example, the user holding the applicator along the palm of his/her hand and squeezing the applicator between a finger and thumb of that hand in the activation region **128.** The proximal portion **124** may also include vent **150,** seal ring **152,** and attachment feature **154.**

Referring to **FIG. 4****,** frangible ampoule **160** is located within hollow body **120** such that at least a portion of the frangible ampoule **160** is located in the activation region **128** so that when the activation region **128** is compressed the frangible ampoule **160** is fractured. The frangible ampoule **160** may be hermetically sealed and can contain a solution. The frangible ampoule **160** may desirably act as a protective barrier to contact between the solution contained therein and the outer environment. For example, the frangible ampoule **160** may be resistant to environmental changes encountered by the applicator **100** during manufacture, transport, and use of the applicator, such as, for example, the vacuum and chemicals, *e.g*., ethylene oxide, that the applicator may be exposed to during a sterilization process, conditions that could damage or destroy the solution inside the frangible ampoule **160.**

Commonly, the frangible ampoule **160** may be formed of a brittle material, such as, for example, a glass. Suitable brittle materials for forming the frangible ampoule **160** include, for example, a sodalime glass, a borosilicate glass, an onion skin borosilicate glass, a polymer, a ceramic, and combinations thereof. Such materials are desirably brittle and will fracture when compressed. This is in contrast to relatively flexible materials that would deform when compressed but which must be punctured to release the substance inside. Typically, the substance contained within the frangible ampoule **160** is released by applying external force to the applicator **100** in the activation region **128** sufficient to shatter the frangible ampoule **160.** In some embodiments, score lines or other features that provide local areas of weakness in the brittle material may be included to control breaking and/or reduce the force required to break the frangible ampoule **160.**

The size and shape of the frangible ampoule **160** is selected to be compatible with the dimensions of the hollow body **120** and the desired volume of substance to be applied. For example, for use in preparation for a small surgical procedure, the amount of substance in the ampoule **160** should generally be sufficient to cover an area of, such as, for example, about 10 square centimeters. For larger surgical procedures, the amount of substance in the ampoule **160** may need to be sufficient to cover at least the torso of a large person, such as, for example, at least about 500-600 square centimeters. The size of the frangible ampoule **160** may be selected to be appropriate for the volume of substance to be delivered in order to minimize product costs and waste. In addition, smaller frangible ampoules **160** may result in products with longer shelf lives. As a nonlimiting example, it has been found that solutions containing cyanoacrylate adhesives have longer shelf lives in small vials compared to larger vials even when the vials are flushed with an inert gas before sealing. Without being bound by theory, it is believed that the smaller interior surface area of a smaller frangible ampoule has fewer sites on the interior surface of the glass that can initiate polymerization of the cyanoacrylate, thereby extending the shelf life of the product.

In some embodiments, the frangible ampoule **160** is selected to apply a substance to a surface having an area such as, for example, about 5 cm² to about 200 square cm². In some embodiments, use of a smaller frangible ampoule **160** permits a frangible ampoule **160** having a thinner wall to be used, thus increasing the ease with which a frangible ampoule **160** may be fractured. In some embodiments, the frangible ampoule **160** is configured to contain, for example, from about 0.2 mL to about 2 mL (*e.g.,* 1 mL) of a substance.

The frangible ampoule **160** contains the substance to be dispensed. Generally, a wide variety of substances can be contained within the frangible ampoule **160,** with the selection of the substance influencing the selection of the materials used to construct the frangible ampoule and other parts of the applicator **100,** as would be readily understood by one of ordinary skill in the art. Substances contained in the frangible ampoule may include, for example, liquids, gels, suspensions, and pastes. In some embodiments, the substance contained in the ampoule **160** is a liquid composition that includes a volatile carrier and an active agent, such as, for example, an antiseptic agent or a pharmaceutical agent. In some embodiments, the applicator**100** may be particularly useful in dispensing substances having viscosities at room temperature of less than about 10,000 cps. In other embodiments, the applicator may be particularly useful in dispensing substances having viscosities less than about 500 cps.

In some embodiments, the frangible ampoule **160** may contain an antiseptic preparation. Examples of suitable antiseptic preparations include those described in U.S. Pat. No. 4,584,192 and those described in U.S. Pat. No. 4,542,012. Other useful fluids include antiseptic preparations, such as, for example, iodophoric skin tinctures, such as DURAPREP Surgical Solution, commercially available from 3M Company, Saint Paul, Minnesota, USA. In some embodiments, the ampoule **160** may be filled with a composition that includes an antimicrobial agent such as iodine, an iodine complex (such as, for example, iodophors), chlorhexidine, chlorhexidine salts (such as, for example, chlorhexidine digluconate and chlorhexidine diacetate), or combinations thereof. Other exemplary antimicrobial agents include C2-C5 lower alkyl alcohols, fatty acid monoesters of glycerin and propylene glycol, polymers that include a (C12-C22) hydrophobe and a quaternary ammonium group, polyquaternary amines (such as, for example, polyhexamethylene biguanide), quaternary ammonium silanes, silver, silver salts (such as silver chloride), silver oxide and silver sulfadiazine, methyl, ethyl, propyl and butyl parabens, octenidine, peroxides (such as, for example, hydrogen peroxide and benzoyl peroxide), and the like, as well as combinations thereof. In other embodiments, the frangible ampoule may contain, for example, medical preparations such as skin barrier products, adhesion enhancing products, adhesive tape trauma protectants, and pharmaceutical agents such as analgesics. As a nonlimiting example, the frangible ampoule may contain a skin barrier product, such as CAVILON Advanced Skin Protectant, commercially available from 3M Company, Saint Paul, Minnesota, USA. In some embodiments, the contents of the frangible ampoule **160** include a cyanoacrylate monomer.

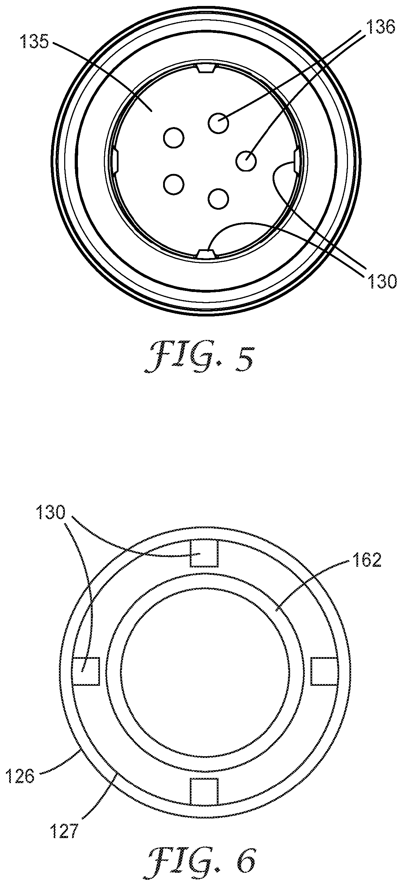

Referring to **FIG. 5****,** the handle **122** of the body **120** includes filter **135,** located between the frangible ampoule **160** and the absorbent component **110.** As shown, the filter **135** may be integrally molded into the handle **122** and is provided with holes **136.** Five holes **136** are shown in **FIG. 5****,** though fewer holes **136,** *e.g.,* 3, or more holes **136,** e.g., 7, are contemplated. The holes 136 are shown having a circular geometry and uniform size, though other geometries, e.g., oval, triangular, rectangular, star, and different sizes for different holes are contemplated. When the frangible ampoule **160** is fractured, the filter **135** prevents shards of the ampoule **160** from reaching the absorbent component **110.** Thus, the filter **135** is made sufficiently thick so as not to be pierced by the shards of the frangible ampoule **160** after the frangible ampoule **160** is broken.

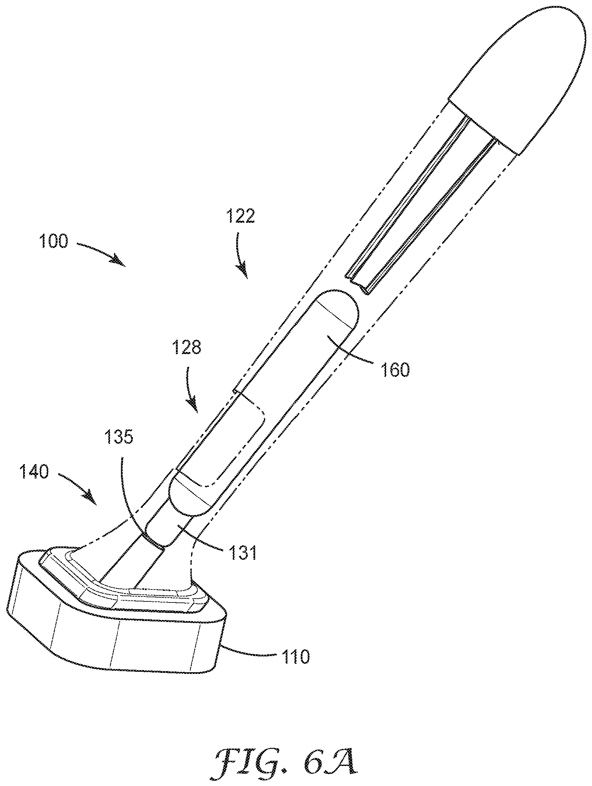

The handle **122** may optionally include one or more ribs **130** on the inner surface **126** of the body **120,** as illustrated in **FIGS. 5** and **6****.** The ribs **130** may extend longitudinally along all or part of the length of the handle from the filter **135** to the proximal portion **124** of the body. When force is applied to activation region **128,** *e.g.,* by the hand of an operator wishing to coat a surface with the solution contained in the frangible ampoule **160,** the ribs **130** act as stress concentrators, thereby decreasing the amount of force required to fracture the wall **162** of the frangible ampoule **160.** The shape of a rib **130** may be selected to optimize its function as a stress concentrator, and may be, for example, approximately square, rectangular, triangular, or semicircular in cross-section. In some embodiments the handle **122** may include one or more ampoule spacers **131** on the inner surface **126** of the body **120** and extending longitudinally from the filter **135** as shown in **FIG. 6a****.** The ampoule spacers **131** are configured to support an end of the ampoule **160** to create a distance (*e.g*., 0.75 cm) between the ampoule **160** and the filter **135.** Creating such a distance, *i.e.,* ampoule space, between the ampoule **160** and the filter **135** may allow for unimpeded flow of contents released from the frangible ampoule **160** after activation. Creating such a distance also allows the activation region **128** to be located further from the distal end **123** of the applicator **100** while still allowing for breaking of the ampoule **160** nearer to an end of the ampoule **160,** thus minimizing the chance of creating large "domes" that can retain contents of the ampoule **160** in the handle **122** after activation.

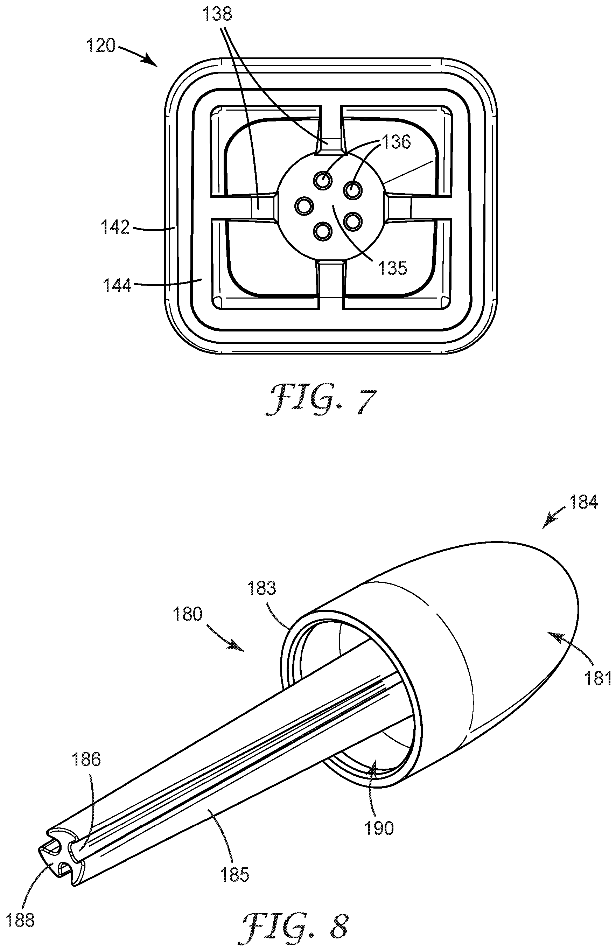

Referring now to **FIG. 7****,** the head **140** may include one or more buttresses **138,** which may extend longitudinally all or a portion of the distance from filter **135** to the distal portion **123** of the body **120.** Buttresses **138** can serve, for example, to improve the rigidity of the head **140** and to direct the flow of a substance released from a fractured frangible ampoule, thereby improving the ease and efficiency with which the applicator **100** is used. The distal ends of the buttresses **138** can also be melted in the hot plate welding process previously described to provide improved attachment of the absorbent component **110.** The buttresses **138** may also function to support the absorbent component **110.**

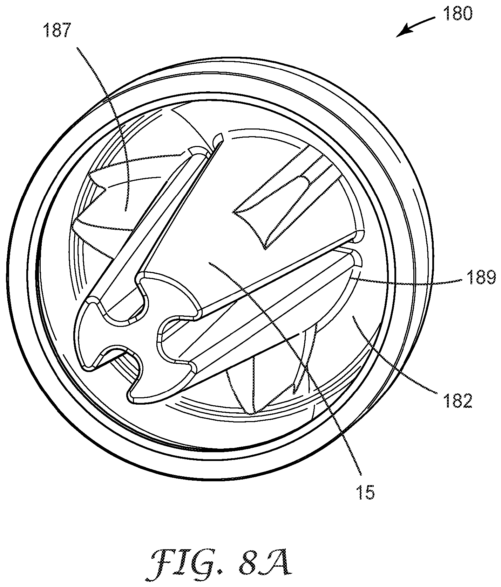

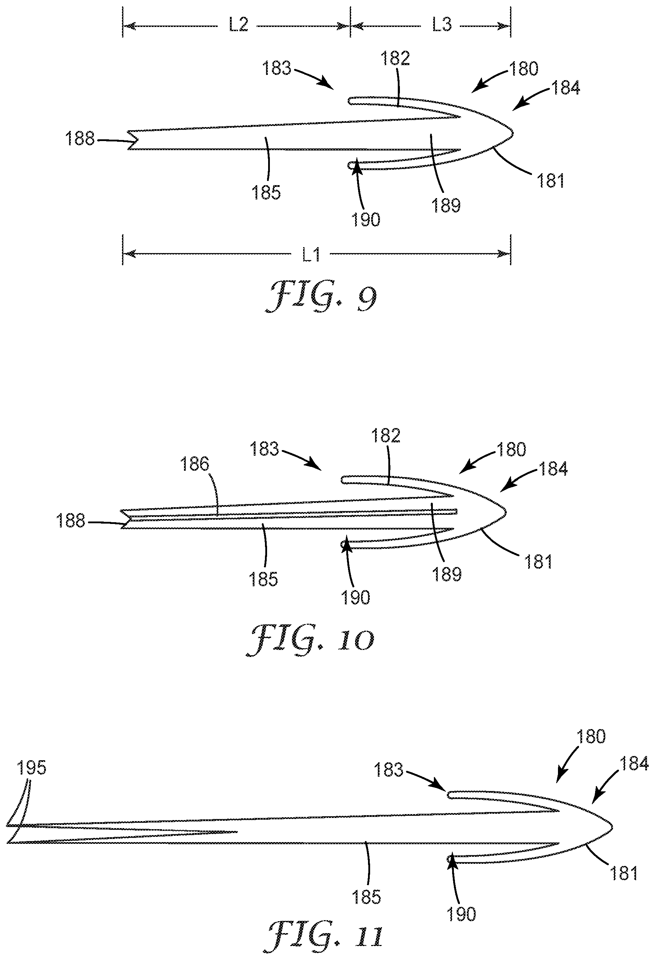

The applicator **100** further comprises cap **180** and spacer **185.** As shown in **FIGS. 8-13****,** the cap **180** has exterior surface **181,** an interior surface **182,** a distal portion **183,** and a proximal portion **184.** As shown in **FIG. 9****,** the cap **180** and spacer **185** combine to create a longitudinal length **L1,** the spacer **185** extending distally beyond the distal end **183** of the cap **180** by a length **L2,** and the cap **180** having a longitudinal length **L3** that extends longitudinally from the most proximal point of the exterior surface **181** to the point at which the exterior surface and interior surface of the cap meet. Spacer **185** has a distal end **188** and a proximal end **189,** the spacer **185** projecting away from the interior surface **182** of the cap **180.** As shown in **FIG. 8a****,** in some embodiments the cap **180** and spacer **185** may include one or more fins **187,** the fins **187** protruding from the proximal end **189** of spacer **185** and joined to a portion of the interior surface **182** of the cap **180.**

As shown in **FIGS. 8-11****,** the spacer **185** projects from the proximal portion **184** of the interior surface **182** of the cap **180,** and the distal end **188** of the spacer **185** extends distally beyond the distal end **183** of the cap **180.** In certain embodiments, the spacer **185** extends distally beyond the distal end **183** of the cap **180** by a length **L2,** as shown in **FIG. 9****.** In some embodiments, the length **L2** is at least 25%, at least 50%, at least 75%, at least 100%, at least 125%, or at least 150% of the length **L3,** *i.e.,* the longitudinal length of the cap **180.** In some embodiments, the length **L2** is less than 500%, less than 400%, less than 300%, less than 200%, or less than 150% *(e.g.,* 140% to 150%) of the length **L3.** In some embodiments, the length **L2** is 50% to 500%, 75% to 400%, 100% to 300%, 125% to 200%, 130% to 170%, or 135% to 160% of the length **L3.**

In some embodiments, length **L1** is at least 50%, at least 75%, at least 90%, or at least 100% of the longitudinal length of frangible ampoule **160.** In some embodiments, length **L1** may be equal to the longitudinal length of frangible ampoule **160.** In some embodiments, length **L1** is less than or equal to 800%, less than or equal to 600%, less than or equal to 400%, or less than or equal to 200% of the longitudinal length of frangible ampoule **160.** In some embodiments, length **L1** is 50% to 800%, 75% to 600%, 90 % to 400%, or 100% to 200% *(e.g.,* 120%) of the longitudinal length of frangible ampoule **160.**

In some embodiments, length **L1** may be at least 10%, at least 20%, at least 30%, at least 40%, or at least 50% of the length of the hollow body **120** as measured along the longitudinal length from filter **135** to the proximal portion **124** of the hollow body **120.** In some embodiments, length **L1** may be less than or equal to 120%, less than or equal to 100%, less than or equal to 90%, less than or equal to 80%, or less than or equal to 70% of the length of the hollow body **120** as measured along the longitudinal length from filter **135** to the proximal portion **124** of the hollow body **120.** In some embodiments, length **L1** may be 10% to 120%, 20% to 100%, 30% to 90%, 40% to 80%, or 50% to 70% *(e.g.,* 67%) of the length of the hollow body **120** as measured along the longitudinal length from filter **135** to the proximal portion **124** of the hollow body **120.**

In some embodiments and as shown in **FIGS. 2****,** **4****,** **8-10****,** **11****,** and **12****,** the distal end **188** of the spacer **185** may have concave curvature. The concave curvature may be selected to complement the convex curvature of an end of an exterior surface of the frangible ampoule **160.** That is, the radius of curvature of the exterior end surface of the frangible ampoule **160** and the radius of curvature of the distal end **188** are approximately equal. Thus, the length of spacer **185** and the curvature of the distal end **188** may be selected to maintain the frangible ampoule in a desired position, such as, for example, maintaining the frangible ampoule in the activation region **128** of the body **120.** The spacer **185** may optionally include a channel **186,** as shown in **FIGS. 2****,** **8****, and** **10****.**

In other embodiments, and as shown in **FIG. 11** the spacer **185** may include fingers **195.** Fingers **195** may be selected to have a length and flexibility sufficient to hold the frangible ampoule **160** in place without having to complement the curvature of the end of the exterior surface of the frangible ampoule **160.** Fingers **195** may be straight as shown or may be curved, such as, for example, in the shape of a helix or spring. As shown in **FIG. 11****,** the spacer **185** may include two fingers **195,** though embodiments including more than two fingers **195** are contemplated. Fingers **195** may be useful, for example, when a particular cap **180,** spacer **185,** and body **120** of an applicator **100** are used with frangible ampoules **160** of varying lengths, shapes, and/or curvatures. In some embodiments, the fingers **195** may be formed from the same material as the spacer **185.** In some embodiments, the fingers **195** may be formed as a unitary structure with the spacer **185.** In some embodiments, the fingers **195** may be constructed using a material that is less rigid, *i.e.,* more flexible, than the material used to form the spacer **185,** and may be joined to the spacer **185** by means known in the art, such as, for example, with an adhesive, a heat seal, a spin weld, an ultrasonic weld, and combinations thereof.

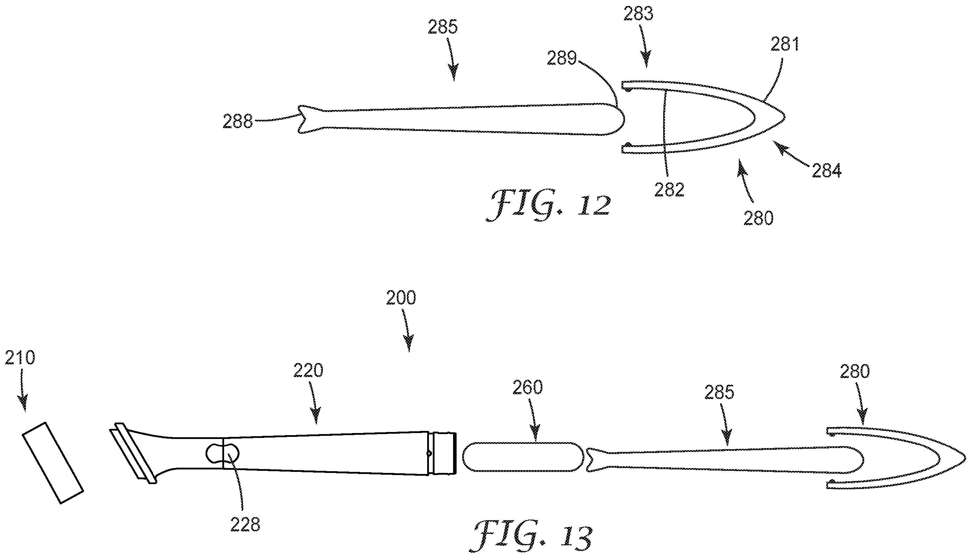

In some embodiments, the cap **180** and the spacer **185** may be formed as a unitary piece, such as by, for example, injection molding. In other embodiments, the cap **180** and the spacer **185** may be formed as separate components which could be joined by means known in the art, such as, for example, with an adhesive, a heat seal, a spin weld, an ultrasonic weld, and combinations thereof, or the cap **180** and the spacer **185** could remain as separate components in the assembled applicator **200.** For example, as shown in **FIGS. 12 and 13****,** a cap **280** has exterior surface **281,** interior surface **282,** distal portion **283,** and proximal portion **284;** and spacer **285** has distal end **288** and proximal end **289.** The distal end **288** of spacer **285** may include a concave curvature, and the proximal end **289** may include a convex curvature. The concave curvature may be selected to complement the convex curvature of an end of an exterior surface of a frangible ampoule. In certain embodiments, the convex curvature of the proximal end **289** is selected to complement the concave curvature of the interior surface **282** of the cap. By selection of length of the spacer **285** and the curvature of its ends, an applicator **200** may be obtained that maintains the frangible ampoule 260 in a desired position in an applicator, *i.e.,* in a position where at least a portion of the frangible ampoule **260** is adjacent to the activation region **228.**

The cap **180** is adapted to be attached to the hollow body **120** by means of a snap fit. Referring to **FIG. 8****,** this is accomplished by providing a retention ring **190** inwardly projecting from side walls adjacent to the major orifice of the cap **180** and which has a smaller inside diameter than the outside diameter of the outwardly projecting ridge of attachment feature **154 (****FIG. 3****)** near the end of the proximal portion of the body. When the applicator is assembled, the retention ring **190** of the cap is snapped over the outwardly projecting ridge of attachment feature **154** on the body. Referring again to **FIG. 3****,** a seal ring **152** may be optionally provided to prevent leakage of the contents of the applicator after the frangible ampoule is fractured.

In order to avoid creating a vacuum and restricting flow through the applicator, a means of maintaining atmospheric pressure in the device is preferably employed. One or more vents in the body functions to aspirate air into the internal volume of the applicator as the substance flows into the absorbent component to maintain atmospheric pressure within the device and prevent "air locking." As shown in **FIG. 3****,** vent **150** may be a hole located in the proximal portion of body **120.** Preferably, the geometry and location of the vent would not result in leakage of substance from the device. To prevent an applicator from leaking, it has been found that a particularly advantageous location for a vent hole is in a location where a portion of the vent hole is covered by the distal end of the cap **180,** as shown in **FIG.1****.**

Referring to **FIG. 2****,** in an embodiment, an applicator **100** may be assembled by attaching an absorbent component **110** to a hollow body **120** using any of the methods previously described; placing a frangible ampoule **160** in the hollow body **120;** and attaching cap **180** with integral spacer **185.** Referring to **FIG. 12****,** in another embodiment, an applicator **200** may be assembled by attaching an absorbent component **210** to a hollow body **220;** placing a frangible ampoule **260** in the hollow body; placing a spacer **285** in the hollow body; and attaching cap **280.**

Generally, the applicators of the present disclosure can be made by known methods. In some embodiments, injection molding may be used. A variety of materials may be used to form the applicators. In some embodiments, applicators formed of low flexural modulus materials such as low-density polyethylene may be used. For example, in some embodiments, materials having a flexural modulus of no greater than 500 Mpa, *e.g.,* no greater than 400 MPa, or even no greater than 350 MPa may be used.

Applicator bodies **120** may be molded from polymers such as, for example, polyethylenes, linear low density polyethylenes, medium density polyethylenes, high density polyethylenes, branched polyethylenes, polypropylenes, and combinations thereof. Applicator bodies of the present disclosure were molded from a combination of linear low-density polyethylene and medium density polyethylene and used to make applicators of the present disclosure.

Applicator caps **180** and spacers **185** may be molded from polymers such as, for example, polyethylenes, linear low-density polyethylenes, medium-density polyethylenes, high-density polyethylenes, branched polyethylenes, polypropylenes, and combinations thereof. Caps including spacers, as shown in **FIG. 7****,** may be molded from high-density polyethylene and used to make applicators **100** of the present disclosure.

### Method of Use of the Applicator

A substance, such as those described above, may be applied to a surface with an applicator as described in the present disclosure by releasing the substance from the frangible ampoule, e.g., the user holding the applicator along the palm of his/her hand and squeezing the applicator between a finger and thumb of that hand in the activation region such that the substance flows into the absorbent component when the frangible ampoule is fractured, and then applying the substance to a surface by pressing the absorbent component to the surface.

In some embodiments, the surface comprises mammalian tissue such as, for example, skin, bone, cartilage, enamel, dentin, cementum, dental pulp, gum tissue, mucous membrane, organ tissue, epithelial tissue, ocular tissue, tympanic tissue, connective tissue, muscular tissue, nervous tissue, and combinations thereof. In some embodiments, the mammalian tissue may include a perforation such as, for example, incision, an abrasion, a laceration, a puncture, an orifice, and combinations thereof.

Various modifications and alterations of the applicator **100** of the present disclosure will become apparent to those skilled in the art without departing from the scope of this disclosure.
The preceding description, given in order to enable one of ordinary skill in the art to practice the claimed invention, is not to be construed as limiting the scope of the disclosure, which is defined by the claims.

## Claims

1. An applicator (100) for applying a substance to a surface and having a distal end configured to be directed toward the surface to be treated and a proximal end extends away from the surface and toward the user, the applicator (100) comprising:
a hollow body (120), wherein the hollow body includes a distal portion (123), a proximal portion (124), an exterior surface (127), an interior surface (126), an activation region (128), and a filter (135);
a frangible ampoule (160), wherein the frangible ampoule contains the substance;
a cap (180), wherein the cap includes a proximal portion (184), a distal portion (183), an exterior surface (181), and an interior surface (182); and
a spacer (185), wherein the spacer includes a distal end (188) and a proximal end (189),
wherein the frangible ampoule is located in the hollow body between the filter and the distal end of the spacer, wherein the filter is located distal to the frangible ampoule such that the filter is located toward the distal end of the applicator and the surface to be treated, wherein the spacer is located between the frangible ampoule and the cap, wherein at least a portion of the frangible ampoule is located in the activation region, and
wherein the activation region further comprises one or more ribs (130) extending from the interior surface of the hollow body, the activation region being configured and arranged such that application of a force to the activation region by the user allows for fracture of the frangible ampoule and the one or more ribs being configured and arranged to concentrate stress to fracture of the frangible ampoule.

2. The applicator of claim 1, wherein the hollow body (120) includes a vent (150).

3. The applicator of any one of the preceding claims, wherein the frangible ampoule (160) abuts the filter (135);
or
wherein the interior surface (126) of the hollow body (120) further comprises an ampoule spacer (131), the ampoule spacer being configured to support an end of the ampoule (160) to create a distance between the ampoule and the filter (135).

4. The applicator of any one of the preceding claims, wherein the longitudinal length of the spacer (185) and cap (180) **L1** is at least 50% of the longitudinal length of the frangible ampoule (160).

5. The applicator of any one of the preceding claims, wherein the spacer (185) extends distally beyond the distal portion (183) of the cap (180) by a length **L2,** wherein **L2** is 50% to 500% of the longitudinal cap length **L3.**

6. The applicator of any one of the preceding claims, wherein the longitudinal length of the spacer (185) and cap (180) **L1** is 50% to 800% of the longitudinal length of frangible ampoule (160).

7. The applicator of any one of the preceding claims, wherein the cap (180) covers a portion of the vent (150).

8. The applicator of any one of the preceding claims, wherein the spacer (185) includes a channel (186).

9. The applicator of any one of the preceding claims, wherein the distal end (188) of the spacer (185) abuts the frangible ampoule (160).

10. The applicator of any one of the preceding claims, wherein the proximal end (189) of the spacer (185) has convex curvature and abuts the interior surface (182) of the proximal portion (184) of the cap (180).

11. The applicator of any one of the preceding claims, wherein the proximal end (189) of the spacer (185) is joined to the interior surface (182) of the cap (180).

12. The applicator of any one of the preceding claims, wherein the substance is selected from the group consisting of a liquid, a gel, a suspension, a paste, and combinations thereof.

13. The applicator of any one of the preceding claims, wherein the applicator (100) further comprises an absorbent component (110), wherein the filter (135) is located between the absorbent component and the frangible ampoule (160).

## Patentansprüche

1. Ein Applikator (100) zum Applizieren einer Substanz auf eine Oberfläche und aufweisend ein distales Ende, das konfiguriert ist, um auf die zu behandelnde Oberfläche gerichtet zu werden, und ein proximales Ende, das sich von der Oberfläche weg und in Richtung des Benutzers erstreckt, der Applikator (100) aufweisend:
einen Hohlkörper (120), wobei der Hohlkörper einen distalen Abschnitt (123), einen proximalen Abschnitt (124), eine Außenoberfläche (127), eine Innenoberfläche (126), einen Aktivierungsbereich (128) und einen Filter (135) einschließt;
eine zerbrechliche Ampulle (160), wobei die zerbrechliche Ampulle die Substanz enthält;
eine Kappe (180), wobei die Kappe einen proximalen Abschnitt (184), einen distalen Abschnitt (183), eine Außenoberfläche (181) und eine Innenoberfläche (182) einschließt; und
ein Abstandsstück (185), wobei das Abstandsstück ein distales Ende (188) und ein proximales Ende (189) einschließt,
wobei sich die zerbrechliche Ampulle in dem Hohlkörper zwischen dem Filter und dem distalen Ende des Abstandsstücks befindet, wobei sich der Filter distal zu der zerbrechlichen Ampulle derart befindet, dass sich der Filter in Richtung des distalen Endes des Applikators und der zu behandelnden Oberfläche befindet, wobei sich das Abstandsstück zwischen der zerbrechlichen Ampulle und der Kappe befindet, wobei sich mindestens ein Abschnitt der zerbrechlichen Ampulle in dem Aktivierungsbereich befindet, und
wobei der Aktivierungsbereich ferner eine oder mehrere Rippen (130) aufweist, die sich von der Innenoberfläche des Hohlkörpers erstrecken, wobei der Aktivierungsbereich derart konfiguriert und angeordnet ist, dass eine Applikation einer Kraft auf den Aktivierungsbereich durch den Benutzer ein Brechen der zerbrechlichen Ampulle ermöglicht, und wobei die eine oder die mehreren Rippen konfiguriert und angeordnet sind, um die Spannung zu konzentrieren, um die zerbrechliche Ampulle zu brechen.

2. Der Applikator nach Anspruch 1, wobei der Hohlkörper (120) eine Entlüftungsöffnung (150) einschließt.

3. Der Applikator nach einem der vorstehenden Ansprüche, wobei die zerbrechliche Ampulle (160) an den Filter (135) angrenzt;
oder
wobei die Innenoberfläche (126) des Hohlkörpers (120) ferner ein Ampullenabstandsstück (131) aufweist, wobei das Ampullenabstandsstück konfiguriert ist, um ein Ende der Ampulle (160) zu stützen, um einen Abstand zwischen der Ampulle und dem Filter (135) zu erzeugen.

4. Der Applikator nach einem der vorstehenden Ansprüche, wobei die Längslänge des Abstandstücks (185) und der Kappe (180) **L1** mindestens 50 % der Längslänge der zerbrechlichen Ampulle (160) beträgt.

5. Der Applikator nach einem der vorstehenden Ansprüche, wobei sich das Abstandsstück (185) distal über den distalen Abschnitt (183) der Kappe (180) um eine Länge **L2** hinaus erstreckt, wobei **L2** 50 % bis 500 % der Kappenlängslänge **L3** beträgt.

6. Der Applikator nach einem der vorstehenden Ansprüche, wobei die Längslänge des Abstandsstücks (185) und der Kappe (180) **L1** 50 % bis 800 % der Längslänge der zerbrechlichen Ampulle (160) beträgt.

7. Der Applikator nach einem der vorstehenden Ansprüche, wobei die Kappe (180) einen Abschnitt der Entlüftungsöffnung (150) abdeckt.

8. Der Applikator nach einem der vorstehenden Ansprüche, wobei das Abstandsstück (185) einen Kanal (186) einschließt.

9. Der Applikator nach einem der vorstehenden Ansprüche, wobei das distale Ende (188) des Abstandsstücks (185) an der zerbrechlichen Ampulle (160) anliegt.

10. Der Applikator nach einem der vorstehenden Ansprüche, wobei das proximale Ende (189) des Abstandsstücks (185) eine konvexe Krümmung aufweist und an der Innenoberfläche (182) des proximalen Abschnitts (184) der Kappe (180) anliegt.

11. Der Applikator nach einem der vorstehenden Ansprüche, wobei das proximale Ende (189) des Abstandsstücks (185) mit der Innenoberfläche (182) der Kappe (180) verbunden ist.

12. Der Applikator nach einem der vorstehenden Ansprüche, wobei die Substanz aus der Gruppe ausgewählt ist, bestehend aus einer Flüssigkeit, einem Gel, einer Suspension, einer Paste und Kombinationen davon.

13. Der Applikator nach einem der vorstehenden Ansprüche, wobei der Applikator (100) ferner eine absorbierende Komponente (110) aufweist, wobei sich der Filter (135) zwischen der absorbierenden Komponente und der zerbrechlichen Ampulle (160) befindet.

## Revendications

1. Applicateur (100) permettant d'appliquer une substance sur une surface et ayant une extrémité distale conçue pour être dirigée vers la surface à traiter et une extrémité proximale s'étend à l'écart de la surface et en direction de l'utilisateur, l'applicateur (100) comprenant :
un corps creux (120), dans lequel le corps creux comporte une partie distale (123), une partie proximale (124), une surface extérieure (127), une surface intérieure (126), une région d'activation (128) et un filtre (135) ;
une ampoule cassable (160), dans lequel l'ampoule cassable contient la substance ;
un capuchon (180), dans lequel le capuchon comporte une partie proximale (184), une partie distale (183), une surface extérieure (181) et une surface intérieure (182) ; et
un élément d'espacement (185), dans lequel l'élément d'espacement comporte une extrémité distale (188) et une extrémité proximale (189),
dans lequel l'ampoule cassable est localisée dans le corps creux entre le filtre et l'extrémité distale de l'élément d'espacement, dans lequel le filtre est localisé distal par rapport à l'ampoule cassable de telle sorte que le filtre est localisé en direction de l'extrémité distale de l'applicateur et de la surface à traiter, dans lequel l'élément d'espacement est localisé entre l'ampoule cassable et le capuchon, dans lequel au moins une partie de l'ampoule cassable est localisée dans la région d'activation, et
dans lequel la région d'activation comprend en outre une ou plusieurs nervures (130) s'étendant à partir de la surface intérieure du corps creux, la région d'activation étant conçue et agencée de telle sorte que l'application d'une force sur la région d'activation par l'utilisateur permet une cassure de l'ampoule cassable et la ou les nervures étant conçues et agencées pour concentrer une contrainte pour casser l'ampoule cassable.

2. Applicateur selon la revendication 1, dans lequel le corps creux (120) comporte un évent (150).

3. Applicateur selon l'une quelconque des revendications précédentes, dans lequel l'ampoule cassable (160) vient en butée contre le filtre (135) ;
ou
dans lequel la surface intérieure (126) du corps creux (120) comprend en outre un élément d'espacement d'ampoule (131), l'élément d'espacement d'ampoule étant conçu pour supporter une extrémité de l'ampoule (160) pour créer une distance entre l'ampoule et le filtre (135).

4. Applicateur selon l'une quelconque des revendications précédentes, dans lequel la longueur longitudinale de l'élément d'espacement (185) et du capuchon (180) **L1** vaut au moins 50 % de la longueur longitudinale de l'ampoule cassable (160).

5. Applicateur selon l'une quelconque des revendications précédentes, dans lequel l'élément d'espacement (185) s'étend distalement au-delà de la partie distale (183) du capuchon (180) sur une longueur **L2,** dans lequel **L2** vaut 50 % à 500 % de la longueur longitudinale de capuchon **L3.**

6. Applicateur selon l'une quelconque des revendications précédentes, dans lequel la longueur longitudinale de l'élément d'espacement (185) et du capuchon (180) **L1** vaut 50 % à 800 % de la longueur longitudinale de l'ampoule cassable (160).

7. Applicateur selon l'une quelconque des revendications précédentes, dans lequel le capuchon (180) couvre une partie de l'évent (150).

8. Applicateur selon l'une quelconque des revendications précédentes, dans lequel l'élément d'espacement (185) comporte un canal (186).

9. Applicateur selon l'une quelconque des revendications précédentes, dans lequel l'extrémité distale (188) de l'élément d'espacement (185) vient en butée contre l'ampoule cassable (160).

10. Applicateur selon l'une quelconque des revendications précédentes, dans lequel l'extrémité proximale (189) de l'élément d'espacement (185) a une courbure convexe et vient en butée contre la surface intérieure (182) de la partie proximale (184) du capuchon (180).

11. Applicateur selon l'une quelconque des revendications précédentes, dans lequel l'extrémité proximale (189) de l'élément d'espacement (185) est jointe à la surface intérieure (182) du capuchon (180).

12. Applicateur selon l'une quelconque des revendications précédentes, dans lequel la substance est choisie dans le groupe constitué d'un liquide, d'un gel, d'une suspension, d'une pâte, et de combinaisons de ceux-ci.

13. Applicateur selon l'une quelconque des revendications précédentes, dans lequel l'applicateur (100) comprend en outre un composant absorbant (110), dans lequel le filtre (135) est localisé entre le composant absorbant et l'ampoule cassable (160).
